# FASCICULE DE BREVET EUROPEEN

(11) **EP 0 804 394 B1**
(45) Date de publication et mention de la délivrance du brevet: **19.09.2001**
(21) Numéro de dépôt: 96901408.3
(22) Date de dépôt: 17.01.1996
(51) Int. Cl.: C07C 15/08, C07C 7/14

(54) **PROCEDE DE PRODUCTION DE PARAXYLENE COMPORTANT UNE CRISTALLISATION A HAUTE TEMPERATURE A AU MOINS UN ETAGE ET UNE FUSION PARTIELLE DES CRISTAUX**
VERFAHREN ZUR HERSTELLUNG VON PARAXYLOL, DAS WENIGSTENS EINE KRISTALLISIERUNGSSTUFE AUF HOHER TEMPERATUR UND EINE STUFE ZUM PARTIELLEN SCHMELZEN DER KRISTALLE ENTHAELT
METHOD FOR PRODUCING PARA-XYLENE BY HIGH-TEMPERATURE CRYSTALLISATION IN AT LEAST ONE STAGE AND PARTIAL CRYSTAL FUSION

(30) Priorité: 20.01.1995 FR 9500746
(43) Date de publication de la demande: 05.11.1997
(73) Titulaire: INSTITUT FRANCAIS DU PETROLE, 92506 Rueil-Malmaison (FR)
(72) Inventeur: MacPHERSON, Stuart, R., Piedmont, CA 94611 (US); MIKITENKO, Paul, F-78590 Noisy-le-Roy (FR)
(74) Mandataire: Andreeff, François
(86) Numéro de dépôt international: FR9600078
(87) Numéro de publication internationale: WO9622262

(56) Documents cités:
- FR-A- 2 681 066
- US-A- 5 329 060

## Description

L'invention concerne un nouveau procédé économique de récupération et de purification du paraxylène à partir d'une charge d'hydrocarbures qui contient du paraxylène à une concentration supérieure à celle à l'équilibre thermodynamique. Elle concerne en particulier les procédés qui enrichissent leur effluent en paraxylène à plus de 30%, notamment les procédés qui prévoient au moins une étape d'enrichissement en paraxylène par cristallisation à très basse température par exemple, cette étape de cristallisation étant suivie d'une étape de purification du paraxylène à au moins un étage (US 3 177 265 et US 2 866 833).

L'invention concerne notamment un procédé de préparation et de purification du paraxylène à partir d'une charge d'hydrocarbures aromatiques à 8 atomes de carbone comportant une combinaison d'étapes d'adsorption sélective, de purification par cristallisation à haute température et d'isomérisation telle que décrite dans les brevets de la Demanderesse FR 2,681,066 et US 5 284 992, incorporés comme références.

Elle s'applique notamment à la préparation de paraxylène à très haute pureté envue de la fabrication d'acide téréphtalique pour la synthèse de tissus synthétiques.

La cristallisation a été utilisée commercialement depuis très longtemps pour isoler et purifier le paraxylène, typiquement à partir d'un mélange de xylènes et d'éthylbenzène proche de l'équilibre (coupe C₈ aromatique).

Les coupes C8 aromatiques proviennent habituellement d'une unité de reformage ou d'une unité de production d'éthylène et plus spécialement à partir des réformats par distillation seule ou extraction en combinaison avec une distillation en fonction de la composition de la charge, de la sensibilité aux impuretés des technologies en aval et de l'économie des procédés de récupération.

La composition typique pondérale d'une coupe C8 aromatique est approximativement de 22% de paraxylène, 16 % d'éthylbenzène, 18 % d'orthoxylène et 44% de métaxylène. De très basses températures sont en général requises pour récupérer efficacement par cristallisation le paraxylène à partir d'une coupe C8. Il existe par ailleurs, une limite eutectique qui empêche la récupération complète de tout le paraxylène à partir d'une coupe C₈. Par exemple, dans une unité de cristallisation à basse température, pour une coupe C₈ aromatique contenant 22 % poids de paraxylène, on ne récupère environ que 50 à 65% du paraxylène, le paraxylène restant sera trouvé dans la liqueur mère appauvrie en paraxylène qui peut être introduite dans une unité d'isomérisation. Celle-ci isomérisera le métaxylène, l'orthoxylène et dans certains procédés, l'éthylbenzène et on obtiendra à nouveau un mélange de xylènes proche de l'équilibre contenant 22 % de paraxylène environ. Ce flux recyclé en combinaison avec de la charge fraîche est alors introduit dans le cristalliseur de façon à récupérer plus de paraxylène. De cette façon, les C₈ aromatiques peuvent être recyclés jusqu'à extinction et récupération d'un maximum de paraxylène, avec des sous-produits résultant de l'isomérisation. La. production de ces sous-produits est une insuffisance significative du système puisque chaque fois que des xylènes sont introduits dans l'unité d'isomérisation, une partie d'entre eux est convertie en non-xylènes tel que du toluène. En effet, la chimie de l'isomérisation est très complexe et les réactions principales qui conduisent à des pertes de xylènes sont la disproportionation (dismutation) des xylènes en toluène et triméthylbenzènes, déalkylation des xylènes, et dans certains cas même, la formation de non aromatiques. Pour cela, les rendements totaux d'un ensemble d'unités d'isomérisation et de cristallisation sont typiquement de 60 à 80 % et une grande boucle de recyclage d'isomérisation est nécessaire pour maximiser la récupération du paraxylène.

Dans les années 1970, un autre procédé a été commercialisé pour éviter la limitation eutectique à partir d'une cristallisation à basse température. Ce procédé utilise une adsorption pour séparer le paraxylène à partir d'un mélange de xylènes. L'adsorption permet de récupérer plus de paraxylène à partir d'une coupe C8 aromatique. Ainsi, à partir d'une charge contenant 22 % poids de paraxylène, on peut récupérer approximativement 97 % de celui-ci par adsorption, en laissant environ 1 % de paraxylène dans la liqueur mère. Il est avantageux de récupérer le produit avec une plus grande efficacité, puisque cela entraîne l'utilisation d'une boucle d'isomérisation beaucoup plus petite pour le recyclage complet de la fraction appauvrie en paraxylène. Cela présente plusieurs avantages, notamment un coût d'investissement plus faible sur une nouvelle unité ou sur l'extension d'une unité existante, des rendements globalement plus élevés dus aux pertes réduites en isomérisation, et des coûts opératoires plus faibles liés à la taille de la boucle d'isomérisation.

On note cependant plusieurs inconvénients liés au système de récupération par adsorption du paraxylène : coût d'investissement élevé, difficulté d'obtention du paraxylène à très haute pureté, sensibilité de l'adsorbant aux impuretés de la charge et sensibilité du système de contrôle aux changements de qualité de la charge.

Par ailleurs, il a été proposé un procédé de cristallisation qui utilise deux étages distincts de cristallisation (procédé AMOCO). Comme les clients demandaient une pureté plus élevée de paraxylène, il devenait de plus en plus difficile d'obtenir les puretés requises par une cristallisation unique. Un procédé a donc été développé, qui refondait complètement les cristaux qui étaient isolés à partir du premier étage de cristallisation à très basse température. Après refusion complète, le flux était refroidi à environ - 17°C pour recristalliser le paraxylène à la pureté désirée. Ce procédé était capable de délivrer de hautes puretés (99.5 %+) après lavage. Cependant, il présente l'inconvénient de coûts opératoires et d'investissements plus élevés dûs au coût de l'énergie associée à une refusion complète et ensuite à une recristallisation du paraxylène.

Un procédé de la Demanderesse a récemment été breveté, qui combine notamment une adsorption à une cristallisation ; il s'agit du brevet US 5,284,992 qui décrit une adsorption sélective pour récupérer du paraxylène à partir d'une charge contenant un mélange de xylènes. Le paraxylène est ensuite purifié par au moins une cristallisation à haute température. Il est enseigné qu'il existe une synergie entre l'adsorption et la cristallisation à haute température, due au fait que l'adsorption est un procédé très efficace de récupération du paraxylène à partir des xylènes et que la cristallisation est un moyen très efficace de purifier à un très haut niveau le paraxylène, ce qui constitue un lien idéal entre les deux technologies. De plus, ce procédé souligne le plein avantage d'une boucle d'isomérisation plus petite due à une récupération par passe presque quantitative du paraxylène dans l'étape d'adsorption de l'unité.

Un autre brevet US 5,329,060 enseigne un procédé comprenant une étape d'adsorption sélective d'une charge d'un mélange de xylènes suivie d'une cristallisation à double étage du paraxylène, une à très basse température (- 50 à - 70° C) et l'autre à haute température (0 à - 10° C). Le coût opératoire et d'investissement de ce procédé est d'autant plus élevé qu'il prévoit une cristallisation à très basse température et une fusion complète des cristaux obtenus avant leur recristallisation.

Enfin, un autre brevet US-A-5,060,862 décrit un procédé comprenant une seule cristallisation à très basse température (-60°C) avec les inconvénients mentionnés ci-haut délivrant après séparation d'une liqueur mère, une masse de cristaux relativement petits.
Ces cristaux se développent ensuite dans une zone de développement dans laquelle ils ont suffisamment de temps pour croître jusqu'à ce qu'ils atteignent la taille moyenne désirée. Par ailleurs, il est précisé que le développement de ces cristaux s'effectue dans une suspension qui est plus riche en cristaux que la suspension qui émerge de la zone de cristallisation à très basse température. Ces deux facteurs (augmentation de la taille et nombre des cristaux) dépendent des températures relatives de la zone de cristallisation et de la zone de développement.
Ce brevet ne suggère donc pas un procédé de production de paraxylène à la plus haute pureté possible et à un coût le plus économique possible.

De plus, dans l'enchaînement d'étapes comprenant une adsorption, une cristallisation et surtout une isomérisation, pour l'obtention de paraxylène très pur, divers types d'impuretés peuvent apparaître dans les différents effluents et entraîner des perturbations dans la marche des unités qui nuisent au rendement obtenu et à la pureté du paraxylène récupéré.

Tout d'abord, lors de l'isomérisation de la fraction appauvrie en paraxylène, des hydrocarbures oléfiniques peuvent être produits en quantité variable suivant les valeurs des pressions partielles d'hydrogène introduit. La formation subséquente de polymères avant et/ou dans l'unité d'adsorption peuvent entraîner de sérieux problèmes de circulation à travers l'adsorbant, voire sa destruction. De plus, des hydrocarbures paraffiniques et naphténiques à 8 et 9 atomes de carbone dont la volatilité est comprise entre celle du toluène, solvant de désorption par exemple, et celle des xylènes, sont des produits intermédiaires de la conversion de l'éthylbenzène en xylènes lors de l'isomérisation et leur accumulation peut s'avérer préjudiciable. Par ailleurs, des hydrocarbures aromatiques à 9 atomes de carbone et plus, présents en faible proportion et mal séparés dans des colonnes de distillation peuvent être nuisibles au procédé, tout comme des aldéhydes et des cétones plus lourds que la charge initiale qui se forment lorsque de l'oxygène se trouve accidentellement dissout.

Enfin, un autre problème est lié à la présence de méthanol. Cet alcool est parfois rajouté en faible proportion dans les mélanges de xylènes à cristalliser pour éviter la cocristallisation d'eau et de paraxylène. En effet, les mélanges de C8 aromatiques secs sont particulièrement hygroscopiques et lors du passage dans les centrifugeuses de la suspension de cristaux de paraxylène dans la liqueur mère, de l'eau contenue dans l'air ambiant peut être absorbée dans la liqueur mère et cette eau peut éventuellement cristalliser en liaison avec la température de cette liqueur mère. De plus, certains échangeurs peuvent présenter des fuites et de l'eau peut passer accidentellement dans le mélange à cristalliser.

L'objet de l'invention est de produire du paraxylène à la plus haute pureté possible avec une plus grande flexibilité et à un coût le plus économique possible.

Un autre objet est de remédier aux inconvénients mentionnés.

Un dernier objet est de limiter la teneur des impuretés, notamment dans la section d'adsorption pour chercher à l'optimiser, dans la mesure où l'adsorbant est très sensible aux impuretés de la charge de la zone d'adsorption.

On a donc remarqué qu'en utilisant une cristallisation à un seul étage à haute température, ou avantageusement une cristallisation à plusieurs étages à haute température et de préférence à deux étages suivie d'une fusion partielle des cristaux récupérés, on obtenait un procédé avec une meilleure récupération du paraxylène et en outre très économique d'autant plus que les fluides réfrigérants utilisés sont faciles à mettre en oeuvre. Par ailleurs, le risque d'augmenter la concentration d'impuretés indésirables dans les effluents se trouve minimisé.

Plus précisément, l'invention concerne un procédé de production de paraxylène de très haute pureté à partir d'une charge contenant un mélange d'hydrocarbures aromatiques de 7 à 9 atomes de carbone, dans lequel on fait circuler une partie au moins de la charge dans une zone adaptée à enrichir une première fraction à au moins 30% poids en paraxylène et on purifie au moins une partie de ladite première fraction par au moins une cristallisation (50) (étape a), on récupère des cristaux en suspension dans une liqueur mère (étape b), on sépare les cristaux de la liqueur mère dans au moins une première zone de séparation (52), (étape c),
le procédé étant caractérisé par les étapes suivantes :
d) on remet en suspension lesdits cristaux obtenus dans au moins une zone (55) de remise en suspension et de fusion partielle distincte de la zone de cristallisation, dans laquelle on recycle une partie au moins d'une liqueur de lavage résultant de l'étape suivante (e) et on récupère une suspension de cristaux,
e) on sépare et on lave avec un solvant de lavage approprié les cristaux en suspension de l'étape d) dans au moins une zone (57) de séparation et de lavage et on récupère d'une part des cristaux de paraxylène pur et d'autre part une liqueur de lavage (58) ; et
f) éventuellement on fond (61) complètement lesdits cristaux purs et on recueille un courant (60) liquide de paraxylène fondu.

Le solvant de lavage peut inclure ce produit liquide de paraxylène pur.

Par cristallisation à haute température, on entend une cristallisation dans au moins un cristalliseur pour chaque étage de cristallisation, d'une solution ou suspension de paraxylène, déjà enrichie en paraxylène, qui correspond à ce que la littérature appelle une étape de purification. Par exemple, le brevet US 2,866,833 incorporé comme référence, mentionne une étape de purification du paraxylène à haute température pouvant aller jusqu'à une température de - 34°C.

En réalisant une étape de fusion partielle des cristaux, on élimine en partie les impuretés superficielles, on fond les cristaux de petite taille et on augmente la température des cristaux, ce qui permet un fonctionnement efficace des organes de séparation et de lavage des cristaux de paraxylène mentionnés ci-après, et par conséquent d'atteindre des puretés très élevées.

L'apport de chaleur nécessaire à la fusion partielle peut être réalisé dans la zone de fusion partielle proprement dite et/ou en amont de celle-ci, grâce par exemple au recyclage d'une partie au moins de la liqueur de lavage éventuellement chauffée.

Selon une première variante, la zone d'enrichissement de la première fraction à au moins 30 % poids en paraxylène peut être au moins une zone de cristallisation à très basse température, par exemple inférieure à - 40° C, dite section de récupération (recovery section), comme celle décrite selon le brevet US 2,866,833 ou comme celles décrites selon le brevet US 5,329,061, incorporés comme références et dans laquelle on introduit une charge contenant des hydrocarbures aromatiques à 8 atomes de carbone. Cette zone d'enrichissement délivre une suspension de cristaux qui est séparée dans une zone de séparation et les cristaux récupérés sont fondus et constituent une partie au moins de ladite première fraction à purifier par la suite. De plus, une liqueur mère résultant de la séparation peut être isomérisée dans une zone d'isomérisation et l'isomérat au moins en partie recyclé vers la zone d'enrichissement (section de récupération).

Selon une deuxième variante, la zone d'enrichissement en paraxylène peut être une zone d'adsorption sélective contenant un adsorbant zéolithique, dans laquelle on introduit une charge contenant des hydrocarbures aromatiques à 8 atomes de carbone. On réalise une adsorption sélective de la charge, en présence d'un solvant de désorption, on récupère ladite première fraction enrichie en paraxylène et une seconde fraction appauvrie en paraxylène, on isomérise ladite seconde fraction dans une zone d'isomérisation contenant un catalyseur d'isomérisation dans des conditions adéquates pour produire un isomérat contenant du paraxylène sensiblement à l'équilibre thermodynamique avec des isomères et on recycle au moins en partie l'isomérat vers la zone d'adsorption.

Le solvant de désorption est en règle générale choisi en fonction de la nature de l'adsorption. A titre d'exemple, on peut utiliser le toluène, le paradiéthylbenzène, le difluorobenzène, le diéthyl toluène, ou une alkyltétraline notamment décrites dans les brevets US-4 886 929, US 4 864 069, US 5 057 643 incorporés comme références.

Selon une troisième variante, la zone d'enrichissement en paraxylène peut être une zone de dismutation d'une charge consistant essentiellement en toluène et utilisant du catalyseur sélectivé par du coke ou du silicium selon les brevets US4.117.026, 4.097.543, 4.851.604, 5.173.461, 5.243.117 et 5.321.183 incorporés comme références. L'effluent de dismutation comprenant des xylènes, du benzène et du toluène qui n'a pas réagi, est avantageusement débarrassé par distillation du benzène et du toluène.

Il est avantageux de disposer en sortie de la zone d'enrichissement, c'est-à-dire par exemple de la zone d'adsorption sélective telle que décrite dans le brevet US 5,284,992 de la Demanderesse, d'un effluent contenant plus de 50 % en poids de paraxylène et de préférence de 75 à 98 %.

Une partie au moins de la liqueur mère résultant d'une étape de séparation après la cristallisation à un étage à haute température de la charge d'hydrocarbures peut être recyclée vers la zone d'enrichissement, par exemple vers la zone d'adsorption. Cette étape de séparation peut être réalisée au moyen d'au moins une centrifugeuse ou d'au moins un filtre rotatif qui sont des moyens connus de l'Homme du métier.

De la même façon, l'étape de séparation et de lavage des cristaux qui est réalisée dans une même zone, après l'étape de fusion partielle, peut être effectuée dans au moins une centrifugeuse ou un filtre rotatif. Selon une variante préférée, elle peut être réalisée dans une zone de séparation et de lavage où le solvant de lavage est introduit à contre-courant des cristaux de paraxylène à laver telle que décrite dans les brevets US 4475355, US 4481169 et CH 515730 incorporés comme références. Plus précisément, cette zone de séparation et de lavage peut comprendre au moins une colonne de lavage, comme la colonne NIRO.

La liqueur de lavage en résultant, éventuellement distillée si c'est nécessaire, peut être avantageusement recyclée au moins en partie dans la zone de cristallisation, une partie pouvant être envoyée vers la zone de fusion partielle pour maintenir le taux de cristaux à un niveau adéquat.

Toutefois, lorsque la zone de séparation et de lavage est une centrifugeuse, on peut recycler aussi une partie de la liqueur de lavage dans la zone de séparation en sortie de l'étage de cristallisation, ce qui permet d'avoir une meilleure pureté du paraxylène final.

Selon un autre mode de mise en oeuvre particulièrement avantageux permettant de réduire le coût d'exploitation du procédé, on peut effectuer la purification du paraxylène dans au moins deux étages de cristallisation à haute température T₁ et T₂ et de préférence entre + 10 et-25° C. Dans ce cas, on réalise les étapes a, b, c et d décrites ci-avant, on cristallise une partie au moins de la liqueur mère résultant de l'étape c) dans une seconde zone de cristallisation à une haute température T2 inférieure à la température T1 de la zone de cristallisation de l'étape a), on récupère des seconds cristaux en suspension dans une seconde liqueur mère, on sépare lesdits seconds cristaux de ladite seconde liqueur mère dans une seconde zone de séparation, on remet en suspension les seconds cristaux dans au moins une zone de remise en suspension et de fusion partielle, on récupère une suspension desdits premiers cristaux résultant de l'étape d) et des seconds cristaux, on sépare et on lave les cristaux obtenus dans au moins une zone de séparation et de lavage avec le solvant de lavage, on récupère d'une part des cristaux de paraxylène pur et d'autre part une liqueur de lavage et éventuellement on fond complètement lesdits cristaux purs puis on recueille le courant liquide de paraxylène fondu.

On peut recycler une partie au moins de la liqueur de lavage dans la première zone de cristallisation, une autre partie pouvant être recyclée vers la zone de fusion partielle, qui récupère les cristaux des deux zones de cristallisation, de façon à maintenir dans ce réservoir une concentration en cristaux d'environ 35 % poids par exemple.

Comme il a été dit précédemment, l'étape de séparation et de lavage des cristaux après l'étape de fusion partielle peut être réalisée soit dans au moins une colonne de lavage à contre-courant, soit dans au moins une centrifugeuse ou un filtre rotatif. L'ensemble des cristaux en suspension peut être séparé et lavé dans le même organe ou bien les premiers cristaux en suspension peuvent être séparés et lavés dans au moins une colonne, une centrifugeuse ou un filtre rotatif et les seconds cristaux en suspension peuvent être séparés et lavés dans au moins une colonne, une centrifugeuse ou un filtre rotatif, qui est distinct(e). Dans le cas de lavage par au moins une centrifugeuse ou un filtre rotatif, on peut recycler une partie de la liqueur de lavage dans la première zone de séparation suivant le premier étage de cristallisation et éventuellement une partie de la liqueur de lavage dans la seconde zone de séparation en sortie du second étage de cristallisation.

Selon une caractéristique du procédé comportant de préférence deux étages de cristallisation, une partie au moins de la liqueur mère en sortie de la seconde zone de séparation peut être recyclée vers la zone d'enrichissement, et plus précisément vers la zone d'adsorption sélective ou vers la zone de cristallisation à très basse température.

Il peut être particulièrement avantageux de maintenir une certaine quantité, environ 30 % en poids par exemple, de cristaux dans l'effluent de première cristallisation et dans l'effluent de seconde cristallisation. A cette fin, on peut recycler une partie de la première liqueur mère dans la première zone de cristallisation et une partie de la seconde liqueur mère dans la seconde zone de cristallisation. On contrôle ainsi indépendamment la température et le taux de cristaux dans chaque cristalliseur.

On a remarqué qu'en opérant à une température T1 de première cristallisation comprise avantageusement entre + 10 et - 5° C, et de préférence entre +5 et -1°C et à une température T2 de seconde cristallisation comprise entre - 5 et - 25° C et de préférence entre-9 et - 25° C, on obtenait d'excellents résultats, la température choisie dépendant du type de réfrigérant, de l'optimisation entre les étapes d'enrichissement, par exemple par adsorption, les cristallisations et le lavage final.

Selon une caractéristique du procédé, le lavage final du paraxylène en fin de procédé, réalisé dans au moins une centrifugeuse ou un filtre rotatif ou dans au moins une colonne de lavage à contre-courant, une colonne NIRO par exemple, peut être effectué par une partie du courant liquide de paraxylène pur provenant de l'étape de fusion complète, qui est utilisé comme solvant de lavage.

Selon une autre caractéristique du procédé, le lavage final du paraxylène en fin de procédé, réalisé dans au moins une centrifugeuse ou un filtre rotatif ou dans au moins une colonne de lavage à contre-courant, une colonne NIRO par exemple, peut être effectué par un solvant de lavage qui est un solvant autre que le paraxylène, le toluène, l'hexane ou le pentane par exemple. Dans ce cas, on distille le courant de paraxylène fondu, on récupère d'une part du paraxylène de très haute pureté et d'autre part du solvant de lavage que l'on recycle au moins en partie dans la zone de séparation et de lavage, on distille de plus la liqueur de lavage qui contient du solvant de lavage en faible quantité et on recycle une partie au moins du solvant de lavage ainsi distillé dans la zone de séparation et de lavage.

Lorsqu'on opère avec une colonne de lavage à contre-courant, et un solvant autre que le paraxylène, il peut être avantageux de séparer par un filtre ou une centrifugeuse, la suspension de cristaux de paraxylène issue de cette colonne, avant l'étape de fusion du paraxylène et de recycler le solvant ainsi séparé dans la colonne de lavage.

La liqueur de lavage, après distillation, est alors en général recyclée comme indiqué ci-avant.

La zone de fusion partielle peut être opérée à une température comprise entre 0 et 11° C de façon à fondre par exemple entre 5 et 60 % poids des cristaux.
Elle comprend en général un réservoir recevant les cristaux et des moyens de chauffage, par exemple par de la vapeur.

On peut utiliser une seule zone de fusion partielle, qui récupère les premiers et seconds cristaux.

Cependant, il peut s'avérer préférable de fondre partiellement les premiers et les seconds cristaux qui sont de degré de pureté différent dans deux zones séparées. Dans ce cas, le recyclage de la liqueur de lavage peut être envisagé dans chacune des deux zones pour maintenir le taux de cristaux à un niveau adéquat. Les suspensions en résultant peuvent être traitées dans des zones de séparation et de lavage distinctes.

Le procédé selon l'invention permet d'obtenir du paraxylène de très haute pureté.

On a observé cependant qu'il était possible d'améliorer les performances de l'installation en contrôlant à tous les niveaux le taux d'impuretés susceptibles de perturber l'adsorption sélective du paraxylène sur l'absorbant et sa cristallisation, que ce soit au niveau de la charge d'hydrocarbures aromatiques, de l'isomérat et/ou de la liqueur mère recyclée. (Demande de brevet de la Demanderesse n° 94/15.896).

Plus généralement, on peut faire circuler une partie choisie parmi au moins en partie la charge, au moins en partie la liqueur mère, au moins en partie l'isomérat dans au moins une zone de traitement à la terre ou matériau équivalent et l'on récupère un premier effluent que l'on introduit au moins en partie dans la zone d'absorption ou faire la zone de cristallisation à très basse température.

Selon la variante comportant une purification à un étage de cristallisation, on peut faire circuler respectivement la liqueur mère résultant de l'étape de séparation dans au moins une zone de traitement à la terre avant de la recycler dans la zone d'enrichissement, en particulier dans la zone d'adsorption ou dans la zone de cristallisation à très basse température.

Selon la variante comportant une purification à deux étages de cristallisation, c'est la seconde liqueur mère résultant de la séparation des cristaux en sortie de la seconde zone de cristallisation qui est introduite dans le réacteur de traitement à la terre.

Ces traitements à la terre permettent d'éliminer au moins en partie les oléfines créées notamment dans l'étape d'isomérisation et une partie au moins des impuretés lourdes, qui circulent dans la boucle zone d'enrichissement, cristallisation et isomérisation.

### Différentes variantes peuvent être réalisées :

La liqueur mère peut être avantageusement introduite au moins en partie dans une colonne de distillation avantageusement celle en aval de la zone d'isomérisation. Cette colonne traite aussi l'effluent de la zone d'isomérisation et délivre une fraction de tête contenant des composés légers (eau, méthanol, C₇⁻) et une autre fraction contenant un mélange distillé de liqueur mère et d'isomérat que l'on introduit ensuite dans la zone de traitement à la terre.

Une fraction de queue de distillation contenant des composés lourds peut aussi être soutirée de cette colonne de distillation, ce qui permet de diminuer la taille des installations aval.

Une partie de la liqueur mère peut aussi être mélangée à l'effluent quel qu'il soit, sortant de la zone de traitement à la terre, que ce soit l'effluent résultant de la circulation de l'isomérat, de la liqueur mère ou de la charge dans la zone de traitement à la terre ou l'effluent résultant de la circulation dans la zone de traitement à la terre, de ces derniers et de la fraction de distillation contenant ledit mélange distillé de liqueur mère et d'isomérat, avant d'être introduits dans la zone d'adsorption sélective.

L'effluent résultant de ces dernières variantes peut être distillé dans au moins une colonne à distiller (colonne dite rerun) qui délivre une fraction de queue contenant des composés lourds et une fraction de tête qui est introduite dans la zone d'adsorption avec éventuellement une partie de la liqueur mère.

Les conditions d'adsorption ou d'élimination des composés indésirables sur la terre sont, en règle générale, les suivantes :
- Température : 100 à 300° C, de préférence 160 à 230° C
- Vitesse spatiale horaire : 1 à 8, de préférence 1 à 4
   (Volume horaire de charge par volume de terre)
- Type de terre : aluminosilicates naturels activés, par exemple la terre référencée F54 chez ENGELHARD,
- Pression: 3 à 100 bar, de préférence 4 à 20 bar.

La colonne de distillation suivant l'isomérisation a en général les caractéristiques suivantes :
- Pression : 1 à 20 bar, de préférence 3 à 8 bar
- Température en fond : 150 à 280° C, de préférence 200 à 240° C
- Nombre de plateaux : 30 à 80, de préférence 50 à 70.

La colonne à distiller dite rerun située entre la zone de traitement à la terre et la zone d'adsorption sélective a habituellement les caractéristiques suivantes :
- Pression : 1 à 20 bar, de préférence 3 à 8 bar
- Température en fond : 160 à 290° C, de préférence 210 à 250° C
- Nombre de plateaux : 40 à 200, le plus souvent 50 à 90.

Selon une autre caractéristique de l'invention, on peut être amené à contenir la teneur en constituants de volatilité intermédiaire entre celle du solvant de désorption et celle du p-xylène à un niveau tolérable. Dans ce cas, au moins une partie de la liqueur mère peut être purgée avant d'être introduite dans la zone de traitement à la terre.

Il peut aussi être avantageux de purger au moins en partie le solvant de désorption résultant des étapes de distillation de la fraction appauvrie ou de la fraction enrichie en paraxylène avant qu'il ne soit recyclé et de compenser la purge du solvant par un apport de solvant frais, soit dans la charge soit en amont de la zone d'adsorption par exemple.

Comme il a été indiqué, il est possible de recycler la liqueur mère de cristallisation en des endroits différents de l'installation selon l'importance des teneurs en composés indésirables mais il peut être avantageux de combiner entre eux ces différents recyclages, par exemple, lorsqu'il s'agit de réutiliser des équipements existants pour la distillation de l'isomérat, le traitement à la terre ou la distillation dite de rerun et lorsque l'un de ces équipements est déjà opéré à son débit maximum.

On peut aussi combiner ces différents recyclages et ces purges lorsqu'on cherche à faire baisser la teneur en une impureté dans la boucle sans chercher à l'éliminer totalement.

L'invention sera mieux comprise au vu des figures suivantes illustrant de manière non limitative plusieurs modes de mise en oeuvre de l'invention, parmi lesquels :
- la figure 1 représente de manière schématique le procédé, combinant une adsorption, une cristallisation et une isomérisation, ainsi que des traitements amont de la liqueur mère, de la charge et de l'isomérat,
- les figures 2 et 3 illustrent la purification du paraxylène par une cristallisation à un seul étage à haute température comprenant respectivement un lavage par du paraxylène fondu et du toluène, et
- la figure 4 montre la purification du paraxylène par un double étage de cristallisation à haute température.

A titre d'exemple, la zone d'enrichissement en paraxylène consiste en une zone d'adsorption sélective sur un adsorbant dans laquelle on fait circuler la charge d'hydrocarbures aromatiques à 8 atomes de carbone.

Les conditions opératoires de l'adsorption en lit mobile simulé (contre courant par exemple) sont choisies de façon que la première fraction contenant le métaxylène, l'orthoxylène et l'éthylbenzène soit un raffinat et la deuxième fraction contenant essentiellement le paraxylène soit un extrait. Ces conditions sont décrites dans le brevet US 5 284 992 incorporé comme référence.

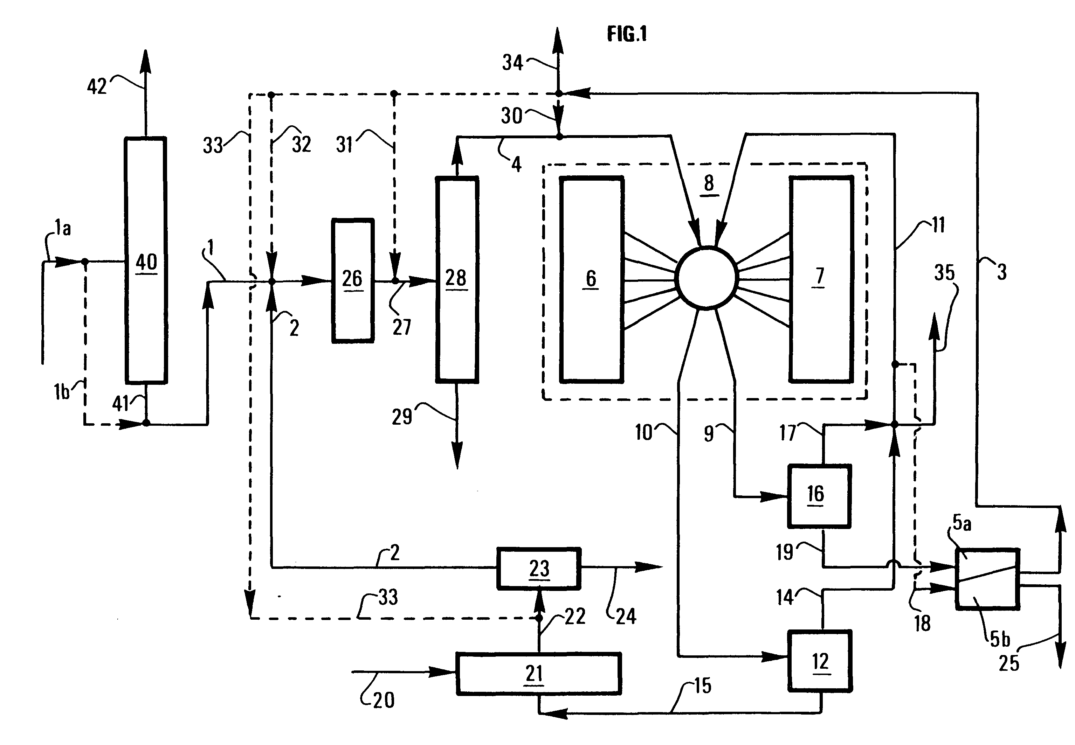

On véhicule (figure 1) par une ligne 1 une charge comprenant environ 20% d'éthylbenzène, 18% de paraxylène, 45% de métaxylène et 17% d'orthoxylène. On y joint par une ligne 2 un effluent recyclé dont la teneur en éthylbenzène est sensiblement plus faible, typiquement 8 à 13% et qui contient des impuretés. Par des lignes 3 et 30, on introduit un autre effluent recyclé dont la teneur en paraxylène est plus forte, typiquement 25 à 45%. Une ligne 4 récupère la charge et ces deux effluents, elle véhicule un mélange de composition approximative, paraxylène 20 à 22,5, éthylbenzène 9 à 14%, orthoxylène 20 à 22,5%, métaxylène 45 à 50% qui est introduit dans une zone d'adsorption 8 en contre-courant simulé comprenant une ou plusieurs colonnes 6 et/ou 7 remplies d'un adsorbant zéolitique, chacune des colonnes étant divisée en un nombre limité de lits, le nombre des lits de chaque colonne pouvant être compris entre 4 et 20, la productivité exprimée par rapport au paraxylène produit étant d'environ 0,07 m³ par m³ de tamis et par heure exprimée aux conditions ambiantes. On désorbe par du toluène, à raison d'environ 1,45 m³ de toluène par m³ de charge, la température opératoire étant à peu près 160°C. On soutire de cette unité par une ligne 10 un raffinat appauvri en paraxylène contenant essentiellement du toluène, du métaxylène, de l'éthylbenzène et de l'orthoxylène et par une ligne 9 un extrait de composition enrichie en paraxylène contenant essentiellement du toluène et du paraxylène, l'impureté majeure étant l'éthylbenzène. Le raffinat est introduit dans une colonne à distiller 12 (température de tête 125°C, température de fond 160°C par exemple). On soutire en tête par une ligne 14 du toluène (environ 30 % de la quantité introduite dans l'adsorption par exemple) contenant par exemple, moins de 2 % de composés C₈ aromatiques et l'on soutire en fond de cette colonne par une ligne 15 un liquide (raffinat débarrassé du solvant) riche en éthylbenzène, en métaxylène et en orthoxylène et appauvri en paraxylène (moins de 3 % par exemple) que l'on envoie dans une unité d'isomérisation 21. Ce raffinat est mis en contact avec de l'hydrogène introduit par une ligne 20 et avec un catalyseur à base de mordénite et de platine sur alumine à environ 380°C. Une ligne 22 conduit l'isomérat de la sortie du réacteur vers un ballon de séparation des constituants gazeux (non représenté sur la figure), puis vers une colonne à distiller 23 (température de tête 90°C, température de fond 160°C par exemple). On soutire en tête par une ligne 24 des hydrocarbures de C₁ à C₅, de l'hexane, du cyclohexane, du benzène et du toluène et en fond de cette colonne par une ligne 2, un effluent contenant 8 à 13% d'éthylbenzène, 21 à 24% de paraxylène, 21 à 24% d'orthoxylène, de 45 à 50% de métaxylène et des impuretés, qui est recyclé vers la zone d'adsorption 8.

La ligne 9 introduit l'extrait dans une colonne à distiller 16 d'où l'on soutire en tête du toluène à moins de 2 % de composés C₈ aromatiques (environ 70 % de la quantité introduite dans l'adsorption par exemple) qui est recyclé par les lignes 17 et 11 vers l'alimentation en solvant de désorption de l'unité d'adsorption. En fond de colonne 16 à environ 160°C on soutire un courant enrichi en paraxylène (à environ 90% de paraxylène) au moyen d'une ligne 19 qui le conduit dans une unité de cristallisation 5a 5b, à un étage par exemple fonctionnant à environ -10°C, duquel on retire du paraxylène pur par une ligne 25.

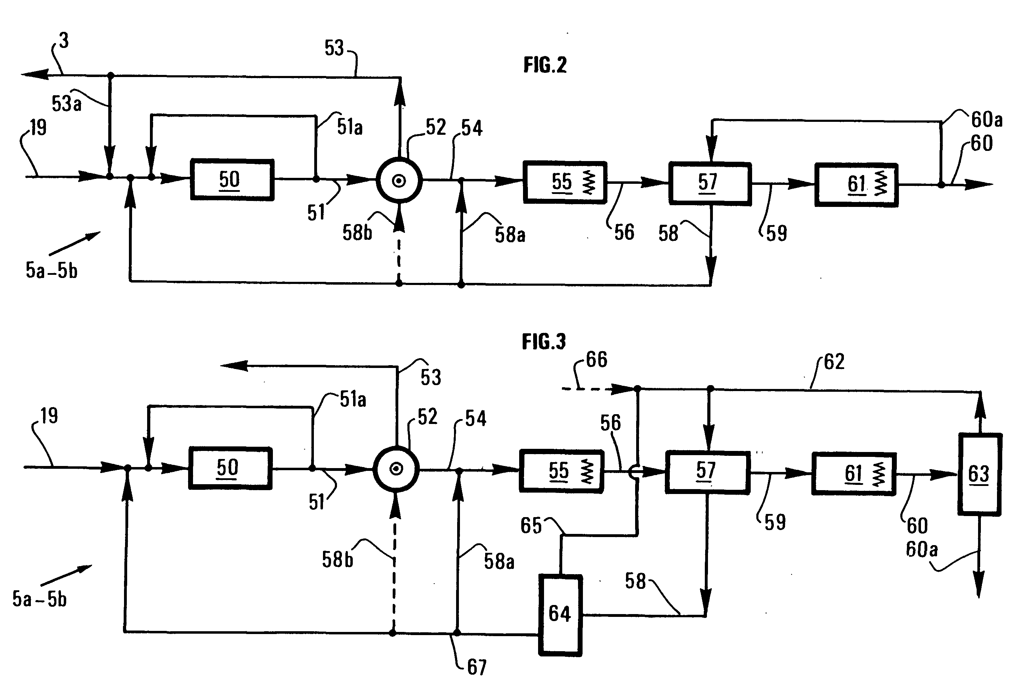

Cette unité de cristallisation ainsi que les moyens de traitement aval sont représentés sur la figure 2.

La charge de cristallisation à 90 % poids par exemple de paraxylène est introduite par la ligne 19 dans au moins un cristalliseur 50 qui comprend en général en amont du cristalliseur proprement dit un bac de charge de cristallisation (non représenté) dans lequel sont introduits la charge et les divers recyclages mentionnés ci-après.

On produit une suspension de cristaux dans une liqueur mère par une ligne 51 qui est en partie recyclée vers le cristalliseur 50 par une ligne 51a, la partie restante étant introduite dans au moins une centrifugeuse 52 ou un filtre rotatif. Une liqueur mère est séparée des cristaux et recyclée au moins en partie par une ligne 53 et une ligne 3 vers l'unité d'adsorption 8. Une partie de cette liqueur mère peut être recyclée dans le bac de charge de cristallisation 50 par une ligne 53a. Les cristaux obtenus imprégnés de liqueur mère sont envoyés par une ligne 54 dans au moins une zone 55 de fusion partielle qui est mise en oeuvre à une température d'environ 10° C. Le taux de cristaux dans le fondeur partiel peut être contrôlé par le recyclage d'une partie d'une liqueur de lavage décrite ci-après et introduite par une ligne 58a et par un apport de calories par tout moyen approprié. On récupère par une ligne 56 une suspension nouvelle de cristaux qui est introduite dans au moins une colonne 57 de lavage type NIRO dans laquelle un solvant de lavage est introduit à contre-courant de l'entrée de la suspension, par une ligne 60a. On récupère en sortie de la colonne des cristaux purs de paraxylène en suspension par une ligne 59 qui sont complètement fondus dans au moins un fondeur 61 lequel délivre par une ligne 60 un courant liquide de paraxylène très pur. Une partie de ce paraxylène liquide peut être introduit comme solvant de lavage dans la colonne 57 par une ligne 60a. On recueille enfin de la colonne 57, par une ligne 58 une liqueur de lavage qui est recyclée au moins en partie dans le cristalliseur 50, une autre partie pouvant être recyclée par une ligne 58a vers le fondeur partiel 55.

Selon une variante de ce dispositif, lorsqu'on utilise, à la place de la colonne 57 de lavage à contre-courant, au moins une centrifugeuse (type pusher par exemple), on préconise en général une centrifugeuse 52 dans laquelle on effectue un lavage des cristaux de la suspension en sortie du cristalliseur 50, au moyen d'une partie de la liqueur de lavage introduite par une ligne 58b en pointillé, qui est recueillie de la centrifugeuse 57.

Selon une autre variante illustrée par la figure 3 avec les mêmes références que celles de la figure 2 et qui utilise un solvant de lavage autre que le paraxylène fondu de haute pureté et plus léger que celui-ci, tel que le toluène, la suspension de cristaux en sortie de la zone de fusion partielle 55 est séparée et lavée avec du toluène introduit par une ligne 66 et du toluène recyclé. En effet, on recueille en sortie du fondeur 61 par la ligne 60 le courant de paraxylène fondu contenant du toluène que l'on distille dans une colonne de distillation 63. On recueille d'une part du paraxylène liquide de très haute pureté par une ligne 60a et d'autre part, par une ligne 62 du toluène que l'on recycle dans la colonne de lavage 57 ou dans la centrifugeuse 57 où s'effectue le lavage. La liqueur de lavage recueillie par la ligne 58 qui contient aussi du solvant est introduite dans une autre colonne de distillation 64, puis recyclée comme décrit précédemment. Le solvant récupéré est aussi recyclé dans la zone de lavage 57 par une ligne 65.

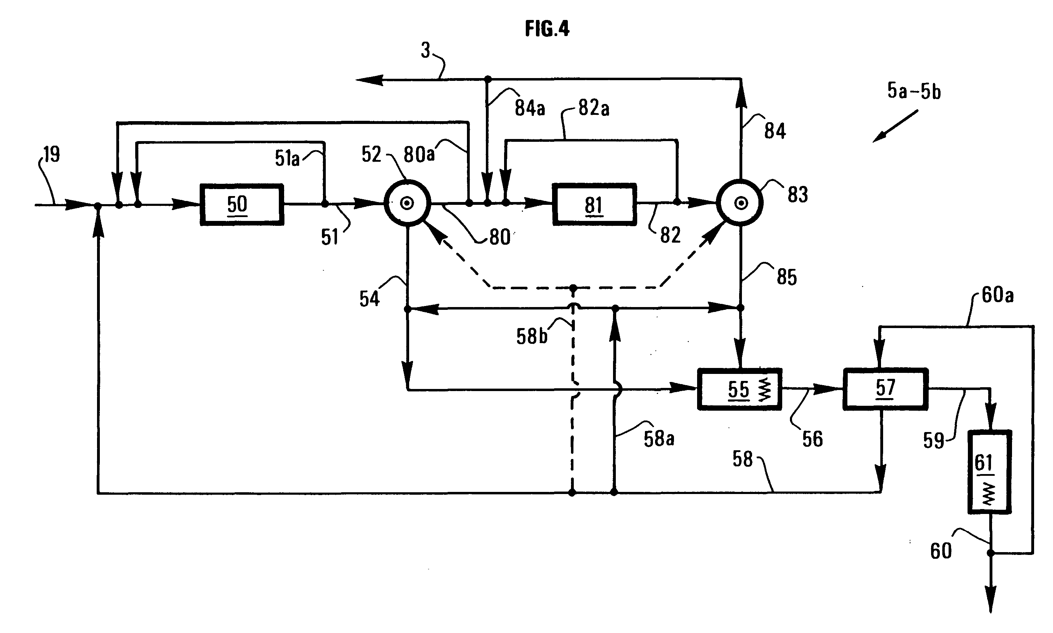

La figure 4 illustre une variante de la purification (zones 5a, 5b) du paraxylène à plusieurs étages de cristallisation. Les mêmes organes que ceux de la figure 2 avec les mêmes fonctions sont référencées avec les mêmes nombres.

La suspension de premiers cristaux en sortie du premier étage de cristallisation 50 dont la température T1 est de 0° C environ est introduite par la ligne 51 dans au moins une centrifugeuse 52. Les premiers cristaux ainsi séparés sont envoyés par la ligne 54 dans la zone de fusion partielle 55 où au moins une partie des plus petits cristaux sont fondus. La liqueur mère en sortie de la centrifugeuse 52 est au moins en partie introduite par une ligne 80 dans au moins un second cristalliseur 81 opérant à une température T2 égale à environ - 18° C. Une partie de cette liqueur mère peut être recyclée par une ligne 80a dans le premier cristalliseur pour y maintenir un taux de cristaux à environ 30 %. Une suspension de seconds cristaux dans une seconde liqueur mère est récupérée par une ligne 82 et introduite au moins en partie dans au moins une seconde centrifugeuse 83, l'autre partie de la suspension étant éventuellement recyclée dans le second cristalliseur 81 par une ligne 82a. On recueille d'une part par une ligne 84 raccordée à la centrifugeuse une seconde liqueur mère que l'on recycle au moins en partie vers la zone d'adsorption 8 par les lignes 84 et 3. Une partie de cette seconde liqueur mère peut être recyclée par une ligne 84a dans le second cristalliseur pour maintenir dans l'effluent 30 % environ de cristaux. On récupère de la centrifugeuse d'autre part, des seconds cristaux imprégnés de liqueur mère par une ligne 85, que l'on introduit dans la zone de fusion partielle 55 qui est, selon la figure 4, commune aux premiers et aux seconds cristaux de paraxylène. L'ensemble des cristaux de petite taille sont généralement fondus et une nouvelle suspension de cristaux (ligne 56) dont le taux de solides à 35 % environ peut être ajusté par une partie de la liqueur de lavage 58a introduite dans les lignes 54 et 85 qui amènent respectivement les premiers et seconds cristaux. Cette nouvelle suspension de cristaux est séparée et lavée comme il a été mentionné ci-avant, par exemple dans la colonne de lavage 57 dans laquelle a été introduit à contre-courant par la ligne 60a du paraxylène fondu de très haute pureté provenant de la zone de fusion complète 61. La liqueur de lavage est recyclée au moins en partie par la ligne 58 et peut être mélangée à la charge de la première cristallisation.

Lorsque la zone de lavage 57 est une centrifugeuse de type pusher par exemple, une partie de la liqueur de lavage peut être recyclée par une ligne 58b en pointillé vers la première 52 et/ou la seconde centrifugeuse 83. On recueille par ailleurs les cristaux de paraxylène par la ligne 59.

Par ailleurs, lorsque le solvant de lavage est un solvant autre que le paraxylène fondu, le dispositif nécessite comme il a été décrit ci-avant (figure 3) une distillation du paraxylène fondu en sortie du fondeur 61 pour obtenir par exemple 99,9 % de pureté et une distillation de la liqueur de lavage 58 permettant de recycler de la liqueur de lavage sensiblement sans solvant dans les diverses lignes de recyclages 58a, 58b et 67 (figures 3 et 4).

Il a été constaté enfin que le lavage à contre-courant dans une colonne était plus avantageux que le lavage dans une centrifugeuse. En effet, à puretés de paraxylène égales le lavage dans une centrifugeuse nécessite des lavages intermédiaires ainsi qu'une étape de fusion partielle à plus haute température, ce qui entraîne la production d'une plus grande quantité de liqueur de lavage à recycler aux cristalliseurs, donc une surface d'échange thermique et une consommation de frigories accrues, ainsi qu'un nombre plus important de centrifugeuses.

Par ailleurs, le procédé incluant un lavage par colonne accepte des charges de compositions globales très variables, 85 à 95 % par exemple de paraxylène en sortie de la zone d'enrichissement, sans des modifications significatives d'équipement.

Comme on l'a schématisé sur la figure 1, la liqueur mère provenant de l'unité de cristallisation 5a, 5b est recyclée vers l'unité d'adsorption 8. Dans le cas d'une cristallisation à deux étages, la liqueur mère provient de l'étage le plus froid de cristallisation après la séparation des cristaux de paraxylène (ligne 84 figure 4). Les impuretés qui circulent dans la boucle du dispositif adsorption, cristallisation, isomérisation peuvent être des hydrocarbures oléfiniques, ainsi que des hydrocarbures paraffiniques et naphténiques ou autres composés oxygénés. Elles peuvent provenir notamment, aussi bien de la charge à traiter qui provient d'un réformage catalytique que de l'isomérisation. Ces impuretés circulent donc et peuvent se retrouver dans toutes les fractions et notamment dans l'extrait et donc dans la liqueur mère résultant de l'étape de cristallisation. Cette liqueur mère peut être introduite par une ligne 32 reliée à la ligne 3 et à la ligne 84 (figure 4) dans au moins un réacteur 26 de traitement à la terre, avantageusement deux, disposé en amont de l'unité d'adsorption et relié par une ligne 27 à celle-ci. A cette ligne 32 peuvent être raccordées la ligne 1 contenant la charge à traiter et la ligne 2 contenant l'isomérat, les trois flux étant ainsi traités en mélange dans le réacteur 26.

Selon une autre variante, la charge 1 peut avoir été prétraitée dans un autre réacteur de traitement à la terre (non indiqué sur la figure). Il en est de même pour l'isomérat 2 qui peut aussi avoir été initialement prétraité après son passage dans l'unité de distillation 23.

Selon une variante préférée, la liqueur mère 3 peut être introduite directement dans la ligne 22 conduisant à l'unité de distillation 23 de l'isomérat, avant d'être traitée, en mélange avec l'isomérat distillé, dans le réacteur 26 de traitement à la terre. Cette variante permet d'éliminer sensiblement tous les composés les plus volatils non seulement de l'isomérat mais de la liqueur mère.

Lorsque l'unité de distillation est réglée pour délivrer aussi, en fond, une fraction supplémentaire contenant la majorité des composés lourds (hydrocarbures en C9+, aldéhydes, cétones), le traitement à la terre du mélange distillé comprenant l'isomérat et la liqueur mère s'en trouve substantiellement amélioré.

Une partie de la liqueur mère peut aussi être recyclée par une ligne 31 à l'effluent 27 du réacteur 26.

L'effluent du réacteur de traitement à la terre et éventuellement la liqueur mère de cristallisation 31 pouvant contenir encore des hydrocarbures lourds tels que des hydrocarbures à neuf atomes de carbone sont introduits par une ligne 27 dans une colonne à distiller 28 qui délivre, en fond de colonne (ligne 29), les impuretés indésirables et en tête un distillat correspondant à la coupe C₈ purifiée, que l'on introduit par la ligne 4 à l'unité d'adsorption 8. Une partie de la liqueur mère peut aussi être introduite dans la ligne 4 par une ligne 30.

Ces divers recyclages peuvent être combinés entre eux, lorsqu'il s'agit, par exemple, de réutiliser des équipements existants pour la distillation 23, le traitement à la terre 26 ou la distillation 28 et lorsque l'un de ces équipements est déjà opéré à son débit maximum ou bien encore lorsque l'on cherche à faire baisser la teneur en une impureté dans la boucle sans chercher à l'éliminer totalement. En d'autres termes, la liqueur mère de l'unité de cristallisation (étage le plus froid), véhiculée par la ligne 3 peut être en partie recyclée vers l'unité d'adsorption 8 soit directement au moyen de la ligne 30, soit indirectement au moyen des lignes 31, 32 ou 33.

De manière à contenir la teneur en constituants de volatilité intermédiaire entre le toluène (solvant de désorption) et les xylènes à un niveau tolérable, par exemple inférieur à 5 %, on effectue au moins une purge de toluène pollué par lesdits constituants au moyen d'une ligne 35 reliée soit à la ligne 17, soit à la ligne 14 soit à la ligne 11 collectant l'ensemble du solvant recyclé vers l'unité d'adsorption 8.

De plus, on peut effectuer une purge d'une partie de la liqueur mère issue de la cristallisation si la teneur en constituants de volatilité intermédiaire y est trop forte. Cette purge est réalisée au moyen de la ligne 34 raccordée à la ligne 3.

La purge de toluène peut être compensée par un appoint de toluène. Étant donné que les sources les plus importantes de coupe C₈ aromatique (ligne 1a) proviennent du reformage catalytique, de la dismutation (disproportionnation) du toluène en benzène et xylène et de la transalkylation toluène - C₉ aromatique et que les effluents de ces unités dont il proviennent sont en partie généralement purifiés dans un train d'unités de distillation dont la colonne 28 peut faire partie, on peut utiliser au moins en partie comme source d'appoint de toluène, soit celui produit en tête (ligne 42) d'une colonne de distillation du toluène 40 en avant du réacteur 26 contenant la terre soit celui qui résulte du by-pass par une ligne 1b d'une partie au moins de la charge (ligne 1a) que l'on mélange à l'effluent 41 de fond de la colonne 40 soit celui qui est introduit dans la charge purifiée (ligne 1) par le déréglage de la colonne 40 qui permet de laisser passer dans la coupe C8 la proportion voulue de toluène.

L'invention est illustrée par l'exemple suivant :

La charge de cristallisation (10 T/h) selon la figure 4 contient 88,5% en poids de paraxylène et est envoyée dans le premier étage de cristallisation et refroidie à 2°C avec un recyclage d'une partie de la liqueur de lavage (2,7 T/h), un recyclage de la suspension de premiers cristaux (127 t/h) et de première liqueur mère (13,1 t/h). Les premiers cristaux obtenus après séparation de la première suspension de cristaux sont mis en suspension avec une partie de la liqueur de lavage (10 T/h) et envoyés vers le bac de fusion partielle. La première liqueur mère est introduite dans le deuxième étage de cristallisation et refroidie à -18°C, en même temps qu'une seconde liqueur mère recyclée (3,7 T/h) et qu'un courant de recyclage de la suspension de seconds cristaux (41,7 T/h). On récupère après centrifugation d'une part un courant de seconde liqueur mère (5,7 T/h) à 43,9% en poids de paraxylène, 2 T/h étant recyclées vers l'adsorption, et d'autre part des seconds cristaux qui sont mis en suspension avec une autre partie de la liqueur de lavage (3,1 T/h) et envoyés vers le bac de fusion partielle, dont la température est maintenue à 7°C.

La suspension de cristaux issue du bac de fusion partielle est lavée dans une colonne de lavage à contre-courant utilisant du paraxylène fondu comme solvant de lavage, de manière à récupérer 15,8 T/h de liqueur de lavage et 8 T/h de paraxylène à 99,9% de pureté.

## Revendications

1. Procédé de production de paraxylène de très haute pureté à partir d'une charge contenant un mélange d'hydrocarbures aromatiques de 7 à 9 atomes de carbone dans lequel on fait circuler une partie au moins de la charge dans une zone adaptée à enrichir une première fraction à au moins 30% poids en paraxylène et on purifie au moins une partie de ladite première fraction par au moins une cristallisation à haute température T1 et avantageusement entre +10 et -25°C dans au moins une zone de cristallisation (50) (étape a), on récupère des cristaux en suspension dans une liqueur mère (étape b), on sépare les cristaux de la liqueur mère dans au moins une première zone de séparation (52), (étape c),
le procédé étant **caractérisé par** les étapes suivantes :
d) on remet en suspension lesdits cristaux obtenus dans au moins une zone (55) de remise en suspension et de fusion partielle distincte de la zone de cristallisation, dans laquelle on recycle une partie au moins d'une liqueur de lavage résultant de l'étape suivante (e) et on récupère une suspension de cristaux,
e) on sépare et on lave avec un solvant de lavage approprié les cristaux en suspension de l'étape d) dans au moins une zone (57) de séparation et de lavage et on récupère d'une part des cristaux de paraxylène pur et d'autre part une liqueur de lavage (58) ; et
f) éventuellement on fond (61) complètement lesdits cristaux purs et on recueille un courant (60) liquide de paraxylène fondu.

2. Procédé selon la revendication 1 dans lequel la zone d'enrichissement en paraxylène est au moins une zone de cristallisation à très basse température dans laquelle est introduite la charge contenant un mélange d'hydrocarbures à 8 atomes de carbone qui délivre, après une étape de séparation, un liquide que l'on isomérise dans une zone d'isomérisation et des cristaux que l'on fond qui sont une partie au moins de ladite première fraction.

3. Procédé selon la revendication 1 dans lequel la zone d'enrichissement en paraxylène est une zone (8) d'adsorption sélective contenant un adsorbant zéolithique dans laquelle est introduite la charge contenant un mélange d'hydrocarbures à 8 atomes de carbone, on réalise une adsorption sélective de la charge, en présence d'un solvant de désorption, on récupère ladite première fraction (9, 19) enrichie en paraxylène et une seconde fraction (10, 15) appauvrie en paraxylène, on isomérise ladite seconde fraction dans une zone (21) d'isomérisation contenant un catalyseur d'isomérisation dans des conditions adéquates pour produire un isomérat plus riche en paraxylène et on recycle (2) au moins en partie l'isomérat vers la zone d'adsorption.

4. Procédé selon l'une des revendications 1 à 3 dans lequel une partie au moins de la liqueur mère (3) résultant de l'étape c) est recyclée vers la zone (8) d'enrichissement.

5. Procédé selon la revendication 1, dans lequel la zone d'enrichissement en paraxylène est au moins une zone de dismutation de la charge consistant essentiellement en toluène produisant un effluent contenant du benzène et des xylènes qu'on distille pour le débarrasser du benzène et du toluène qui n'a pas réagi.

6. Procédé selon l'une des revendications 1 à 3 et 5 dans lequel on cristallise une partie au moins de la liqueur mère résultant de l'étape c) dans une seconde zone (81) de cristallisation à une haute température T2 inférieure à la température T1 de la zone (50) de cristallisation de l'étape a), on récupère des seconds cristaux en suspension dans une seconde liqueur mère, on sépare lesdits seconds cristaux de ladite seconde liqueur mère dans une seconde zone (83) de séparation, on remet en suspension les seconds cristaux dans au moins une zone (55) de remise en suspension et de fusion partielle, on récupère une suspension desdits premiers cristaux résultant de l'étape d) et des seconds cristaux, on sépare et on lave les cristaux en suspension dans au moins une zone (57) de séparation et de lavage avec le solvant de lavage, on récupère d'une part des cristaux de paraxylène pur et d'autre part une liqueur (58) de lavage, et éventuellement on fond (61) complètement lesdits cristaux purs puis on recueille (60) le courant liquide de paraxylène fondu.

7. Procédé selon l'une des revendications 1 à 6 dans lequel on recycle une partie au moins de la liqueur (58) de lavage dans la première zone (50) de cristallisation ou zone de cristallisation à haute température (T1).

8. Procédé selon l'une des revendications 1,2,3,6 à 7 dans lequel une partie au moins de la seconde liqueur mère (84) est recyclée vers la zone d'adsorption (8) ou vers la zone de cristallisation à très basse température.

9. Procédé selon l'une des revendications 6 à 8, dans lequel on recycle une partie de la seconde liqueur mère (84) dans la seconde zone (81) de cristallisation à plus basse température.

10. Procédé selon l'une des revendications 1 à 9 dans lequel la zone de séparation et de lavage est au moins une colonne de lavage à contre courant dans laquelle on introduit comme solvant de lavage une partie du courant liquide (60a) de paraxylène fondu.

11. Procédé selon l'une des revendications 1 à 9 dans lequel la zone de séparation et de lavage est une colonne de lavage à contre-courant par un solvant autre que le paraxylène purifié, on envoie les cristaux de paraxylène pur en suspension provenant de ladite colonne dans au moins une centrifugeuse ou un filtre avant la fusion complète des cristaux pour séparer le solvant de lavage que l'on recycle dans la colonne.

12. Procédé selon l'une des revendications 1 à 9, dans lequel la zone de séparation et de lavage est au moins une centrifugeuse ou un filtre rotatif, on y introduit comme solvant de lavage une partie du courant liquide (60a) de paraxylène fondu.

13. Procédé selon l'une des revendications 1 à 9 dans lequel le solvant de lavage est un solvant autre que le paraxylène, on distille (63) le courant de paraxylène fondu, on récupère d'une part du paraxylène de très haute pureté et d'autre part du solvant de lavage que l'on recycle (62) au moins en partie dans la zone (57) de séparation et de lavage, on distille (64) de plus la liqueur de lavage et on recycle une partie au moins du solvant de lavage (65) distillé dans la zone (57) de séparation et de lavage.

14. Procédé selon l'une des revendications 1 à 9, 12 et 13 dans lequel la zone (57) de séparation et de lavage est au moins une centrifugeuse ou un filtre rotatif et dans lequel on recycle une partie de la liqueur de lavage dans la première zone (52) de séparation.

15. Procédé selon l'une des revendications 6 à 9, 12 à 14 dans lequel la zone (57) de séparation et de lavage est au moins une centrifugeuse ou un filtre rotatif et dans lequel on recycle une partie de la liqueur de lavage dans la seconde zone (83) de séparation.

16. Procédé selon l'une des revendications 1 à 15, dans lequel la zone de remise en suspension et de fusion partielle des premiers cristaux et la zone de fusion partielle des seconds cristaux sont séparées.

17. Procédé selon l'une des revendications 6 à 16 dans lequel la température de la première zone de cristallisation est de + 10 à - 5°C et la température de la seconde zone de cristallisation de - 5 à - 25°C.

18. Procédé selon l'une des revendications 1 à 17, dans lequel la température de la première zone de séparation est sensiblement constante et sensiblement égale à la température de la première zone de cristallisation.

19. Procédé selon l'une des revendications 1 à 18 dans lequel le recyclage de la liqueur de lavage vers la zone de remise en suspension et de fusion partielle est effectué en amont de la dite zone.

20. Procédé selon l'une des revendications 1 à 19 dans lequel ledit recyclage est chauffé.

21. Procédé selon l'une des revendications 1 à 20 dans lequel la zone de remise en suspension et de fusion partielle est opérée à une température comprise entre 0 et 11°C.

22. Procédé selon l'une des revendications 1 à 21 dans lequel ladite zone de remise en suspension et de fusion partielle comprend des moyens de chauffage.

23. Procédé selon l'une des revendications 1 à 22 dans lequel la suspension de l'étape d) contient environ 35 % de solides.

## Claims

1. Process for the production of paraxylene of very high purity from a charge containing a mixture of aromatic hydrocarbons having 7 to 9 carbon atoms in which at least a part of the charge is made to circulate in a zone suited to enrich a first fraction to at least 30% by weight of paraxylene, and at least a portion of said first fraction is purified by at least one high-temperature crystallization T₁ and advantageously between + 10°C and -25°C in at least one crystallization zone, (50) (step a), a slurry of crystals in a mother liquor is recovered (step b), the crystals are separated from the mother liquor in at least one first separation zone (52) (step c), the process being **characterized by** the following steps :
d) reslurrying said crystals obtained in at least one reslurrying and partial melting zone separate from the crystallization zone, where at least a part of a washing liquor resulting from the following step (e) is recycled and a slurry (suspension) of crystals is recovered,
e) the crystals in suspension of step (d) are separated and washed with a suitable washing solvent in at least one separating and washing zone (57), and, on the one hand, pure paraxylene crystals, and, on the other hand, a washing liquor (58) are recovered; and
f) said pure crystals are optionally completely melted (61) and a liquid stream (60) of melted paraxylene is collected.

2. Process according to claim 1, wherein the paraxylene-enrichment zone is at least one crystallization zone at very low temperature in which the charge containing a mixture of hydrocarbons having 8 carbon atoms is introduced which delivers, after a separation step, a liquid that is isomerized in an isomerization zone and crystals that are melted which are at least a portion of said first fraction.

3. Process according to claim 1, wherein the paraxylene-enrichment zone is a selective adsorption zone (8) containing a zeolitic adsorbent in which the charge containing a mixture of hydrocarbons having 8 carbon atoms is introduced, a selective adsorption of the charge is carried out, in the presence of a desorption solvent, said first paraxylene-enriched fraction (9, 19) and a second paraxylene-depleted fraction (10, 15) are recovered, said second fraction is isomerized in an isomerization zone (21) containing a catalyst for isomerization under conditions suitable for producing an isomerate that is richer in paraxylene and the isomerate is recycled (2) at least in part to the adsorption zone.

4. Process according to one of claims 1 to 3, wherein at least a portion of mother liquor (3) resulting from step c) is recycled to enrichment zone (8).

5. Process according to claim 1, wherein the paraxylene enrichment zone is at least a zone of disproportionation of the charge consisting essentially in toluene, that produces an effluent containing benzene and xylenes, which is distilled to remove benzene and unreacted toluene.

6. Process according to one of claims 1 to 3 and 5. wherein at least a portion of the mother liquor resulting from step c) is crystallized in a second crystallization zone (81) at a high temperature T2 that is lower than temperature T1 of crystallization zone (50) of step a), second crystals in suspension are recovered in a second mother liquor, said second crystals of said second mother liquor are separated in a second separation zone (83), the second crystals are reslurried in at least one reslurrying and partial melting zone (55), a suspension of said first crystals resulting from step d) and second crystals are recovered, the crystals in suspension are separated and washed in at least one zone (57) for separating and washing with the washing solvent, pure paraxylene crystals, on the one hand, and a washing liquor (58), on the other hand, are recovered, and optionally said pure crystals are completely melted (61), then the liquid stream of melted paraxylene is collected (60).

7. Process according to one of claims 1 to 6, wherein at least a portion of washing liquor (58) is recycled in first crystallization zone (50) or high-temperature crystallization zone (T1).

8. Process according to one of claims 1,2,3,6 to 7, wherein at least a portion of second mother liquor (84) is recycled to the adsorption zone (8) or to very low temperature crystallization zone.

9. Process according to one of claims 6 to 8, wherein a portion of second mother liquor (84) is recycled in second crystallization zone (81) at lower temperature.

10. Process according to one of claims 1 to 9, wherein the zone for separating and washing is at least one countercurrent washing column into which at least a portion of liquid stream (60a) of melted paraxylene is introduced as washing solvent.

11. Process according to one of claims 1 to 9, wherein the zone for separating and washing is a column for countercurrent washing by a solvent other than purified paraxylene, the pure paraxylene crystals in suspension coming from said column are sent into at least one centrifuge or one filter before the complete melting of the crystals to separate the washing solvent that is recycled in the column.

12. Process according to one of claims 1 to 9, wherein the zone for separating and washing is at least one centrifuge or one rotary filter, a portion of liquid stream (60a) of melted paraxylene is introduced there as washing solvent.

13. Process according to one of claims 1 to 9, wherein the washing solvent is a solvent other than paraxylene, the stream of melted paraxylene is distilled (63), on the one hand, liquid paraxylene of very high purity and, on the other hand, washing liquor are recovered and are recycled (62), at least in part, in zone (57) for separating and washing, the washing liquor is also distilled (64) and at least a portion of distilled washing solvent (65) is recycled in zone (57) for separating and washing.

14. Process according to one of claims 1 to 9, 12 and 13, wherein zone (57) for separating and washing is at least one centrifuge or one rotary filter and wherein a portion of the washing liquor is recycled in first separation zone (52).

15. Process according to one of claims 6 to 9, 12 to 14, wherein zone (57) for separating and washing is at least one centrifuge or one rotary filter and wherein a portion of the washing liquor is recycled in second separation zone (83).

16. Process according to one of claims 1 to 9, wherein the reslurrying and partial melting zone of the first crystals and the reslurrying and partial melting zone of the second crystals are separate.

17. Process according to one of claims 6 to 16, wherein the temperature of the first crystallization zone is from +10 to -5°C and the temperature of the second crystallization zone from -5 to -25°C.

18. Process according to one of claims 1 to 17, wherein the temperature of the first separation zone is approximately constant and approximately equal to the temperature of the first crystallization zone.

19. Process according to one of claims 1 to 18, wherein the recycling of the washing liquor to the reslurrying and partial melting zone is carried out upstream of said zone.

20. Process according to one of claims 1 to 19, wherein said recycling is heated.

21. Process according to one of claims 1 to 20, wherein the reslurrying and partial melting zone is operated at a temperature comprised between 0 and 11°C.

22. Process according to one of claims 1 to 21, wherein said reslurrying and partial melting zone comprises heating means.

23. Process according to one of claims 1 to 21, wherein the slurry of step d) contains about 35% of solids.

## Patentansprüche

1. Verfahren zur Herstellung von Paraxylol sehr hoher Reinheit, ausgehend von einer Charge, die eine Mischung von aromatischen Kohlenwasserstoffen mit 7 bis 9 Kohlenstoffatomen enthält, in dem man einen Teil wenigstens der Charge in einer Zone zirkulieren lässt, die ausgelegt ist, um eine erste Fraktion auf wenigstens 30 Gew.% an Paraxylol anzureichern, und man wenigstens einen Teil dieser ersten Fraktion durch wenigstens eine Kristallisation bei hoher Temperatur T1 und vorteilhaft zwischen +10 und -25°C in wenigstens einer Kristallisationszone (50) (Stufe a) reinigt, man Kristalle in Suspension in einer Mutterlauge gewinnt (Stufe. b), man die Kristalle von der Mutterlauge in wenigstens einer ersten Trennzone (52) (Stufe c) trennt,
wobei das Verfahren durch die folgenden Stufen gekennzeichnet ist:
d) man bringt diese Kristalle wieder in Suspension, die in wenigstens einer Zone (55) des Wieder-in-Suspension-Bringens und des partiellen Schmelzens erhalten werden, welche verschieden von der Kristallisationszone ist, in die man einen Teil wenigstens einer aus der folgenden Stufe (e) resultierenden Waschflüssigkeit rezykliert und man gewinnt eine Kristallsuspension,
e) man trennt und man wäscht die Kristalle in Suspension der Stufe d) mit einem geeigneten Waschlösungsmittel in wenigstens einer Zone (57) der Trennung und des Waschens und man gewinnt einerseits reine Paraxylolkristalle und andererseits eine Waschflüssigkeit (58); und
f) gegebenenfalls schmilzt man diese reinen Kristalle vollständig (61) und man sammelt einen flüssigen Strom (60) von geschmolzenem Paraxylol.

2. Verfahren nach Anspruch 1, in dem die Zone der Anreicherung des Paraxylols wenigstens eine Kristallisationszone bei sehr niedriger Temperatur ist, in der die, eine Mischung von Kohlenwasserstoffen mit 8 Kohlenstoffatomen enthaltende, Charge eingeführt wird, die nach einer Trennstufe eine Flüssigkeit liefert, die man in einer Isomerisationszone isomerisiert, und Kristalle liefert, die man schmilzt und die ein Teil wenigstens dieser ersten Fraktion sind.

3. Verfahren nach Anspruch 1, in dem die Zone der Anreicherung von Paraxylol eine Zone (8) der selektiven Adsorption ist, welche ein zeolitisches Adsorptionsmittel enthält, in der die Charge, die eine Mischung von Kohlenwasserstoffen mit 8 Kohlenstoffatomen enthält, eingeführt wird, man eine selektive Adsorption der Charge in Gegenwart eines Desorptionslösungsmittels verwirklicht, man diese erste an Paraxylol angereicherte Fraktion (9,19) und eine zweite an Paraxylol verarmte Fraktion (10,15) gewinnt, man diese zweite Fraktion in einer, einen Isomerisierungskatalysator enthaltenden, Isomerisierungszone (21) unter geeigneten Bedingungen isomerisiert, um ein an Paraxylol reicheres Isomerat herzustellen und man wenigstens teilweise das Isomerat zu einer Adsorptionszone rezykliert (2).

4. Verfahren nach einem der Ansprüche 1 bis 3, in dem ein Teil wenigstens der Mutterlauge (3), die aus der Stufe c) resultiert, zur Anreicherungszone (8) rezykliert wird.

5. Verfahren nach Anspruch 1, in dem die Anreicherungszone an Paraxylol wenigstens eine Zone der Dismutation der im wesentlichen aus Toluol bestehenden Charge ist, die einen Benzol und Xylole enthaltenden Abstrom erzeugt, den man destilliert, um ihn von Benzol und Toluol, das nicht reagiert hat, zu befreien.

6. Verfahren nach einem der Ansprüche 1 bis 3 und 5, in dem man einen Teil wenigstens der aus der Stufe c) resultieren Mutterlauge in einer zweiten Kristallisationszone (81) bei einer hohen Temperatur T2 kristallisiert, die kleiner als die Temperatur T1 der Kristallisationszone (50) der Stufe a) ist, man zweite Kristalle in Suspension in einer zweiten Mutterlauge gewinnt, man diese zweiten Kristalle von der zweiten Mutterlauge in einer zweiten Trennzone (83) trennt, man die zweiten Kristalle in wenigstens einer Zone (55) des Wieder-in-Suspension-Bringens und des partiellen Schmelzens wieder in Suspension bringt, man eine Suspension dieser ersten aus der Stufe d) resultierenden Kristalle und zweite Kristalle gewinnt, man die Kristalle in Suspension in wenigstens einer Zone (57) der Trennung und des Waschens mit einem Waschlösungsmittel trennt und wäscht, man einerseits reine Paraxylolkristalle und andererseits eine Waschflüssigkeit (58) gewinnt und man gegebenenfalls vollständig diese reinen Kristalle schmilzt und dann den flüssigem Strom (60) geschmolznen Paraxylols sammelt.

7. Verfahren nach einem der Ansprüche 1 bis 6, in dem man einen Teil wenigstens der Waschflüssigkeit (58) in die erste Kristallisationszone (50) oder Kristallisationszone bei hoher Temperatur (T1) rezykliert.

8. Verfahren nach einem der Ansprüche 1, 2, 3, 6 bis 7, in dem wenigstens ein Teil der zweiten Mutterlauge (84) zu der Adsorptionszone (8) oder zu der Kristallisationszone bei sehr niedriger Temperatur rezykliert wird.

9. Verfahren nach einem der Ansprüche 6 bis 8, in dem man einen Teil der zweiten Mutterlauge (84) in die zweite Kristallisationszone (81) bei sehr niedrigerer Temperatur rezykliert.

10. Verfahren nach einem der Ansprüche 1 bis 9, in dem die Zone der Trennung oder des Waschens wenigstens eine Waschkolonne bei Gegenstrom ist, in der man als Waschlösungsmittel einen Teil des flüssigen Stromes (60a) von geschmolzenem Paraxylol einführt.

11. Verfahren nach einem der Ansprüche 1 bis 9, in dem die Zone der Trennung und des Waschens eine Waschkolonne bei Gegenstrom durch wenigstens ein anderes Lösungsmittel als gereinigtes Paraxylol ist, man die reinen Paraxylolkristalle, die von dieser Kolonne kommen, in wenigstens eine Zentrifuge oder einen Filter vor dem vollständigen Schmelzen der Kristalle schickt, um das Waschlösungsmittel, das man in die Kolonne rezykliert, zu trennen.

12. Verfahren nach einem der Ansprüche 1 bis 9, in dem die Zone des Trennens und Waschens wenigstens eine Zentrifuge oder in Drehfilter ist und man dort als Waschlösungsmittel einen Teil des flüssigen Stromes (60a) von geschmolzenem Paraxylol einführt.

13. Verfahren nach einem der Ansprüche 1 bis 9, in dem das Waschlösungsmittel ein anderes Lösungsmittel als Paraxylol ist, man den Strom von geschmolzenem Paraxylol destilliert (63), man einerseits Paraxylol sehr hoher Reinheit und andererseits Waschlösungsmittel gewinnt, das man wenigstens teilweise in die Zone (57) der Trennung und des Waschens rezykliert (62), man zusätzlich die Waschflüssigkeit destilliert (64) und man einen Teil wenigstens des destillierten Waschlösungsmittels (65) in die Zone (57) der Trennung und des Waschens rezykliert.

14. Verfahren nach einem der Ansprüche 1 bis 9, 12 und 13, in dem die Zone (57) der Trennung und des Waschens wenigstens eine Zentrifuge oder ein Drehfilter ist und in der man einen Teil der Waschflüssigkeit in die erste Trennzone (52) rezykliert.

15. Verfahren nach einem der Ansprüche 6 bis 9, 12, bis 14, in dem die Zone (57) der Trennung und des Waschens wenigstens eine Zentrifuge oder ein Drehfilter ist und in dem man einen Teil der Waschflüssigkeit in die zweite Trennzone (83) rezykliert.

16. Verfahren nach einem der Ansprüche 1 bis 15, in dem die Zone des Wieder-in-Suspension-Bringens und des partiellen Schmelzens der ersten Kristalle und die Zone des partiellen Schmelzens der zweiten Kristalle getrennt sind.

17. Verfahren nach einem der Ansprüche 6 bis 16, in dem die Temperatur der ersten Kristallisationszone von +10 bis -5°C ist und die Temperatur der zweiten Kristallisationszone von -5 bis -25°C ist.

18. Verfahren nach einem der Ansprüche 1 bis 17, in dem die Temperatur der ersten Trennzone im wesentlichen konstant und im wesentlichen gleich der Temperatur der ersten Kristallisationszone ist.

19. Verfahren nach einem der Ansprüche 1 bis 18, in dem die Rezyklierung der Waschflüssigkeit zu der Zone des Wieder-in-Suspension-Bringens und des partiellen Schmelzens stromaufwärts dieser Zone vorgenommen wird.

20. Verfahren nach einem der Ansprüche 1 bis 19, in dem die Rezyklierung erwärmt wird.

21. Verfahren nach einem der Ansprüche 1 bis 20, in dem die Zone des Wieder-in-Suspension-Bringens und des partiellen Schmelzens bei einer Temperatur, die zwischen 0 und 11°C liegt, betrieben wird.

22. Verfahren nach einem der Ansprüche 1 bis 21, in dem die Zone des Wieder-in-Suspension-Bringens und des partiellen Schmelzens Heizmittel umfasst.

23. Verfahren nach einem der Ansprüche 1 bis 22, in dem die Suspension der Stufe d) etwa 35% Feststoffe enthält.
